# FASCICULE DE BREVET EUROPEEN

(11) **EP 3 532 033 B1**
(45) Date de publication et mention de la délivrance du brevet: **05.08.2020**
(21) Numéro de dépôt: 17804606.6
(22) Date de dépôt: 25.10.2017
(51) Int. Cl.: A61K 9/08, A61K 47/18, A61K 47/20, A61K 31/573

(54) **SOLUTION PHARMACEUTIQUE D'HYDROCORTISONE POUR DISPOSITIF D'INJECTION**
PHARMAZEUTISCHE HYDROCORTISONLÖSUNG FÜR EINE INJEKTIONSVORRICHTUNG
PHARMACEUTICAL HYDROCORTISONE SOLUTION FOR AN INJECTION DEVICE

(30) Priorité: 26.10.2016 FR 1660393
(43) Date de publication de la demande: 04.09.2019
(73) Titulaire: Crossject, 21000 Dijon (FR)
(72) Inventeur: BARDONNAUD, Pauline, 21000 Dijon (FR); FLORES-IVANEZ, Frédérique, 21200 Ruffey Les Beaune (FR)
(74) Mandataire: Agasse, Stéphane
(86) Numéro de dépôt international: PCT/FR2017/052941
(87) Numéro de publication internationale: WO 2018/078285

(56) Documents cités:
- WO-A1-01/78810
- WO-A1-2015/092758
- Efcortesol Injection, Amdipharm UK Ltd.: "Efcortesol Injection", Authorisation number PL 20072/0229, 2009, pages 1-10, XP055375071, Extrait de l'Internet: URL:http://www.mhra.gov.uk/home/groups/spc pil/documents/spcpil/con1492496656049.pdf [extrait le 2017-05-22]

## Description

L'invention concerne une solution pharmaceutique d'hydrocortisone ou d'un sel pharmaceutiquement acceptable de celle-ci (ci-après abrégée « solution pharmaceutique d'hydrocortisone ») destinée à être injectée par voie parentérale, notamment intramusculaire.

L'hydrocortisone et les sels d'hydrocortisone sont connus pour être utilisés comme agents anti-inflammatoires. Il est courant d'employer ces substances actives dans le traitement notamment de l'asthme, des désordres endocriniens, des désordres dermatologiques et des états allergiques, ainsi que de l'insuffisance surrénalienne aigüe.

L'hydrocortisone et ses sels appartiennent à la famille des corticostéroïdes.

L'hydrocortisone, ainsi que ses sels sont sensibles à l'oxydation. C'est pourquoi, les solutions pharmaceutiques de ces substances actives comprennent toujours au moins un antioxydant. Cependant, les antioxydants mis en œuvre dans ces solutions pharmaceutiques sont eux-même instables.

C'est pourquoi, il est connu que ces solutions pharmaceutiques comprennent un 2^{ième} antioxydant qui est de l'éthylène-diamine-tétra-acétate disodique (ci-après abrégé « EDTA disodique »). L'EDTA disodique a pour fonction de stabiliser (ou autrement dit conserver) l'antioxydant qui est lui-même choisi pour conserver l'hydrocortisone en la préservant donc de l'oxydation.

Par exemple, on connaît le produit pharmaceutique commercialisé par la société AMDIPHARM sous la dénomination commerciale Efcortesol® qui est une solution pharmaceutique de phosphate de sodium d'hydrocortisone à une concentration de 13,39% m/v (autrement dit il y a 13,39 g de ce sel dans 100 mL de solution). Ce produit est conditionné soit dans des ampoules de 1 mL qui contiennent 100 mg d'hydrocortisone, soit dans des ampoules de 5 mL qui contiennent 500 mg d'hydrocortisone.

L'Efcortesol® comprend en outre :
- deux antioxydants : l'EDTA disodique qui a également pour fonction d'être un agent chélatant et le sodium formaldéhyde bisulfite qui est aussi un anti-microbien, ainsi que de
- de l'hydrogénophosphate de sodium, du dihydrogénophosphate de sodium, de l'acide phosphorique (concentré à 10% en volume),
- de l'eau pour préparation injectable.

Cependant, le sodium formaldéhyde bisulfite ne figure pas sur la GRAS liste (« GRAS » étant l'acronyme anglophone pour « Generally Recognized As Safe » et pouvant se traduire par « liste des substances généralement considérées sans danger ») de la FDA (« FDA » étant l'acronyme anglophone pour « Food and Drug Administration », à savoir l'Autorité américaine de surveillance des aliments et des médicaments).

De plus, cet antioxydant n'est pas décrit dans l'ouvrage de référence internationale en matière de source d'informations sur les excipients pharmaceutiques qu'est le « Handbook of pharmaceutical excipients ».

Par ailleurs, afin de respecter la dose nécessaire à l'indication thérapeutique (par exemple une insuffisance surrénalienne aigüe, de l'asthme ou toute maladie nécessitant la prise rapide et importante d'un corticostéroïde) lors d'une injection intramusculaire qui implique un volume injecté plus faible, une concentration de la solution pharmaceutique plus élevée que celle proposée par le produit pharmaceutique Efcortesol® peut s'imposer.

C'est pourquoi, il serait souhaitable de disposer d'une nouvelle solution pharmaceutique d'hydrocortisone ou d'un sel pharmaceutiquement acceptable de celle-ci qui puisse être plus concentrée que la solution connue d'Efcortesol® et qui soit parfaitement stable au cours du temps. Il serait également avantageux que le profil d'impuretés soit proche de cette solution connue d'Efcortesol® et qu'elle présente, dans la mesure du possible, un minimum d'impuretés inconnues pour lesquelles une caractérisation et une étude de toxicité laborieuse seraient nécessaires.

Les inventeurs de la présente invention ont cherché à mettre au point une nouvelle solution pharmaceutique d'hydrocortisone ou d'un sel pharmaceutiquement acceptable de celle-ci destinée à être injectée par voie parentérale, en particulier intramusculaire, en substituant le sodium formaldéhyde bisulfite par un autre antioxydant, ladite solution devant :
- présenter un niveau d'impuretés au maximum égal, de préférence inférieur, au niveau d'impuretés de l'Efcortesol® pris comme produit pharmaceutique de référence, et
- être dépourvue d'impureté inconnue en quantité importante qui nécessiterait une caractérisation.

Les inventeurs de la présente invention ont découvert de manière tout à fait surprenante que l'association des deux antioxydants que sont la rongalite (aussi connue sous la dénomination de « sodium formaldéhyde sulfoxylate ») et de l'EDTA disodique dans une solution pharmaceutique d'hydrocortisone ou d'un sel pharmaceutiquement acceptable de celle-ci permettait d'atteindre parfaitement ces objectifs.

La présente invention a pour premier objet une solution pharmaceutique d'hydrocortisone qui comprend au moins :
- de l'hydrocortisone ou un sel pharmaceutiquement acceptable de celle-ci ;
- de la rongalite ;
- de l'EDTA disodique ;
- un solvant.

L'association des deux antioxydants que sont l'EDTA disodique et la rongalite confère à la solution d'hydrocortisone une stabilité au cours du temps parfaitement remarquable, qui est équivalente, voire même meilleure que celle du produit de référence Efcortesol ®.

En outre, la rongalite est un excipient parfaitement autorisé par les administrations pharmaceutiques.

Enfin, il a été constaté qu'une solution pharmaceutique selon l'invention présente un niveau d'impuretés proche de celui du produit de référence Efcortesol ®, voire même est inférieur à celui-ci, et ce dans des conditions de stockage (à savoir de température et d'humidité relative) variées et forcées.

La présente invention réside donc dans la sélection d'une association de deux antioxydants spécifiques pour stabiliser une solution pharmaceutique d'hydrocortisone. Ainsi, l'invention concerne l'association de deux antixoxydants que sont l'ETDA disodique et la rongalite pour stabiliser une solution pharmaceutique d'hydrocortisone.

Le sel pharmaceutiquement acceptable de l'hydrocortisone peut être choisi parmi le phosphate de sodium d'hydrocortisone, le sodium succinate d'hydrocortisone, l'hydrogéno-succinate d'hydrocortisone, le butyrate d'hydrocortisone et l'acétate d'hydrocortisone.

De préférence, le sel d'hydrocortisone est le phosphate de sodium d'hydrocortisone.

Le solvant peut être tout solvant pharmaceutiquement acceptable et qui est compatible avec l'hydrocortisone et ses sels, ainsi que tout autre composé que comprend ladite solution pharmaceutique selon l'invention. Il peut s'agir d'eau, en particulier d'eau utilisée dans les dispositifs d'injection (autrement dit de l'eau pour préparation injectable), ainsi que de l'eau contenant des additifs isotonisants ou des solutions de chlorure de sodium. L'eau pour préparation injectable est ultra-pure et exempte de contaminants bactériens.

La solution pharmaceutique peut en outre comprendre au moins un tampon. Par exemple, il peut s'agir d'un tampon choisi parmi l'acétate de sodium, le citrate de sodium, le dihydrogénophosphate de sodium et l'hydrogénophosphate de sodium.

De préférence, le tampon est choisi parmi le dihydrogénophosphate de sodium et l'hydrogénophosphate de sodium.

Dans un mode de réalisation de l'invention, la solution pharmaceutique comprend comme tampons du dihydrogénophosphate de sodium et de l'hydrogénophosphate de sodium.

Le pH de la solution pharmaceutique est avantageusement compris entre 7 et 9, de préférence entre 7,5 et 8,5.

Ladite solution pharmaceutique peut en outre comprendre au moins un excipient pharmaceutique acceptable.

Dans un mode de réalisation de l'invention, la solution pharmaceutique comprend :
- du phosphate de sodium d'hydrocortisone ;
- de la rongalite ;
- de l'EDTA disodique ;
- au moins un tampon ;
- un solvant, de préférence de l'eau pour préparation injectable.

De manière préférée, dans ce mode de réalisation de l'invention, la solution pharmaceutique comprend comme tampons du dihydrogénophosphate de sodium et de l'hydrogénophosphate de sodium.

De manière avantageuse, la concentration en hydrocortisone dans ladite solution pharmaceutique est comprise entre 150 mg/mL et 170 mg/mL.

De manière tout à fait préférée, la concentration en hydrocortisone dans ladite solution est de 160 mg/mL.

La solution pharmaceutique selon l'invention peut comprendre en g pour 200 mL de solution :
- entre 20 g et 60 g, de préférence entre 40 g et 45 g, d'hydrocortisone ou d'un sel pharmaceutiquement acceptable de celle-ci ;
- entre 0,2 g et 1,2 g, de préférence entre 0,3 g et 0,5 g, de rongalite ;
- entre 0,1 g et 0,6 g, de préférence entre 0,15 g et 0,25 g, d'EDTA disodique ;
- entre 160 g et 200 g, de solvant, de préférence d'eau pour préparation injectable.

Ladite solution peut en outre comprendre jusqu'à 10 g d'au moins un excipient pharmaceutiquement acceptable.

La présente invention a aussi pour objet un procédé de préparation de la solution pharmaceutique selon l'invention telle que décrite ci-dessus qui comprend au moins les étapes suivantes :
a) on prépare sous agitation un mélange comprenant au moins le solvant, la rongalite et l'EDTA disodique ;
b) on y ajoute sous agitation de l'hydrocortisone ou un sel pharmaceutiquement acceptable de celle-ci de manière à obtenir ladite solution pharmaceutique ;
c) optionnellement on effectue au moins une étape de filtration sur la solution pharmaceutique obtenue à l'issue de l'étape b).

Si la solution pharmaceutique comprend des tampons, ces tampons sont avantageusement préalablement à l'étape a) mélangés ensemble avant d'être ajoutés au mélange de l'étape a).

Lorsque la solution pharmaceutique comprend au moins un excipient pharmaceutiquement acceptable, cet excipient peut être ajouté au mélange de l'étape a).

Au cours du procédé de préparation, les étapes mettant en œuvre une agitation sont avantageusement réalisées à une vitesse d'agitation comprise entre 200 et 400 tour/minute, plus préférentiellement entre 250 et 300 tour/minute.

L'étape de filtration peut comprendre au moins une filtration choisie parmi la filtration clarifiante et la filtration stérilisante.

De manière préférée, l'étape de filtration consiste en une filtration clarifiante suivie d'une filtration stérilisante.

L'invention a aussi pour objet la solution pharmaceutique telle que décrite ci-dessus pour son utilisation dans le traitement des maladies choisies parmi l'insuffisance surrénalienne aigüe, l'asthme ou toute maladie nécessitant la prise rapide et importante d'un corticostéroïde. De préférence, il s'agit du traitement de l'insuffisance surrénalienne aigüe et de l'asthme.

Ladite solution est avantageusement administrée par voie parentérale, de préférence par voie intramusculaire.

La présente invention a aussi pour objet un kit d'injection, de préférence un kit d'injection par voie intramusculaire, comportant :
- un dispositif d'injection ;
- la solution pharmaceutique selon l'invention telle que décrite ci-dessus.

De manière avantageuse, le volume d'injection du dispositif d'injection est compris entre 0,60 mL et 0,65 mL.

Ledit dispositif d'injection peut être à usage unique. Par exemple, il s'agit d'un tube pré-rempli prêt à l'emploi.

Dans un mode de réalisation préféré de l'invention, ledit dispositif est un dispositif d'injection pré-rempli, à usage unique, sans aiguille et automatique grâce à un générateur de gaz dont il est équipé. Il peut s'agir d'un dispositif d'injection sans aiguille à cartouche pyrotechnique. A cet égard, les demandes de brevet FR 2 815 544 A1 et FR 2 807 946 A1 décrivent un exemple de ce dispositif d'injection.

De manière tout à fait avantageuse, le dispositif d'injection est un dispositif commercialisé par la société Crossject sous la dénomination commerciale ZENEO®.

Ainsi, dans un mode de réalisation du kit d'injection selon l'invention, le dispositif d'injection est un dispositif d'injection sans aiguille à cartouche pyrotechnique.

### PARTIE EXPERIMENTALE :

Des expérimentations ont été réalisées afin de comparer la teneur en impuretés de trois solutions de phosphate de sodium d'hydrocortisone. Deux des solutions étaient des solutions dites « comparatives » (ci-après dénommées « comparative 1 » et « comparative 2 ») et la 3^{ième} solution était une solution selon l'invention » (ci-après dénommée « invention »).

Plus précisément, les solutions comparatives 1 et 2 différaient de la solution invention du fait qu'elles étaient dépourvues de rongalite qui était remplacée respectivement par du formaldehyde sodium bisulfite et du sodium métabisulfite.

La solution comparative 1 comprenait la même association d'antioxydants (c'est-à-dire EDTA disodique et formaldehyde sodium bisulfite) que le produit pharmaceutique Efcortesol ®, à savoir le produit de référence dont la présente invention se propose de présenter un niveau d'impuretés au maximum égal, de préférence inférieur, au niveau d'impuretés de ce produit.

Le tableau 1 ci-dessous détaille pour chacune des 3 solutions préparées les pourcentages massiques exprimés par rapport à la masse totale de la solution considérée de chacun des constituants (à l'exception de l'eau).

**Tableau 1 détaillant les pourcentages massiques des constituants des solutions comparatives 1 et 2 et invention**

| | **Comparative 1** | **Comparative 2** | **Invention** |
|---|---|---|---|
| **phosphate de sodium d'hydrocortisone** | 20,42 | 20,42 | 20,42 |
| **ETDA disodique** | 0,08 | 0,08 | 0,08 |
| **rongalite** | 0 | 0 | 0,16 |
| **formaldehyde sodium bisulfite** | 0,16 | 0 | 0 |
| **métabisulfite** | 0 | 0,16 | 0 |
| **Solution tampon** : eau pour préparation injectable comprenant les tampons : | Qsp 100ml | Qsp 100 ml | Qsp 100 ml |
| - hydrogénophosphate de sodium : | 0,27 | 0,27 | 0,27 |
| - dihydrogénophosphate de sodium : | 0,01 | 0,01 | 0,01 |

Dans le tableau 1, Qsp est l'abréviation de « quantité suffisante pour» signifiant que la quantité de solution tampon est telle que le volume de la solution est complété à 100 mL.

Dans ces solutions, la concentration en hydrocortisone était de 160 mg/mL.

Les solutions comparatives 1 et 2 et invention ont toutes été préparées de la manière suivante :
a) on a préparé sous agitation sous barreau magnétique à 250 tour/minute un mélange contenant du dihydrogénophosphate de sodium et de l'hydrogénophosphate de sodium,
b) on y a ajouté sous agitation sous barreau magnétique à 300 tour/minute l'eau, les deux antioxydants (à savoir l'EDTA disodique et rongalite, formaldehyde sodium bisulfite et métabisulfite pour respectivement les solutions selon l'invention, comparative 1 et comparative 2, et on a poursuivi l'agitation pendant 9 minutes ;
c) on y a ajouté sous agitation sous barreau magnétique à 300 tour/minute du phosphate de sodium d'hydrocortisone et on a poursuivi l'agitation jusqu'à dissolution complète de ce sel de manière à obtenir une solution ;
d) on a effectué une étape de filtration clarifiante à un débit de 250 mL/minute sur la solution obtenue à l'étape c), suivie d'une étape de filtration stérilisante à une pression de 56 Pa à un débit de 140 mL/minute ;
e) on a bullé à l'azote la solution obtenue à l'étape d).

Le bullage à l'azote est un inertage partiel qui permet de diminuer le contact de la solution pharmaceutique avec l'oxygène et donc de limiter l'oxydation de ladite solution.

Les trois solutions ainsi obtenues, c'est-à-dire les solutions comparatives 1,2 et invention ont été conditionnées dans des tubes en verre équipés de deux bouchons à leurs extrémités.

Plus précisément, les tubes ont été remplis de 0,65 mL de solution. Un balayage à l'azote a été appliqué à la surface des solutions pour chasser l'oxygène présent dans l'espace de tête de ces tubes. Enfin, les tubes ont été fermés.

Après un mois de stockage sous une humidité relative de 75% et à des températures de 40°C et 50°C, à partir d'analyses par chromatographie en phase liquide à haute performance (ci-après abrégée « CLHP »), on a déterminé les pourcentages d'aire des impuretés dans chacune des trois solutions.

Ces pourcentages d'aire sont exprimés par rapport à l'aire du pic principal de la solution considérée (à savoir solution comparative 1, et 2 et invention).

Les analyses CLHP ont été réalisées dans les conditions suivantes :
- une colonne commercialisée par la société Phenomenex sous la dénomination commerciale Luna® C18(2) d'épaisseur de 5 µm et de dimensions (250 x 4,6) mm ;
- débit de 1,5 mL/minute ;
- volume d'injection de 5 µL ;
- détecteur ultra-violet à longueur d'onde variable comprise entre 190 et 400 nm ou détecteur photodiode ;
- longueur d'onde de 254 nm ;
- température de la colonne 25°C (+/-) 2°C ;
- échantillon à température ambiante ;
- phase mobile A : 0,1% (v/v) d'acide trifluoroacétique dans eau purifiée ;
- phase mobile B : 0,1% (v/v) d'acide trifluoroacétique dans acétonitrile.

Le tableau 2 ci-dessous détaille la composition de la phase mobile en fonction du temps.

| Temps (minutes) | Phase A (%) | Phase B (%) |
|---|---|---|
| 0 | 85 | 15 |
| 10 | 85 | 15 |
| 22 | 55 | 45 |
| 38 | 30 | 70 |
| 38,1 | 85 | 15 |
| 43 | 85 | 15 |

Le tableau 3 ci-dessous détaille la somme des pourcentages des impuretés et le nombre d'impuretés pour les solutions qui ont été stockées à 40°C pendant 1 mois.

**Tableau 3 détaillant les pourcentages d'aire des impuretés dans les solutions comparatives 1, 2 et invention stockées à 40°C sous 75% d'humidité relative**

| | Comparative 1 | Comparative 2 | Invention |
|---|---|---|---|
| somme des impuretés | 0,58% (6 impuretés) | 0,21% (2 impuretés) | 0,34% (4 impuretés) |

Le profil d'impuretés de la solution selon l'invention est meilleur que celui de la solution comparative 1. En outre, avec la solution selon l'invention, aucune impureté supérieure à 0,2% n'a été détectée.

Le tableau 4 ci-dessous détaille les pourcentages d'aire des impuretés précitées pour les solutions qui ont été stockées à 50°C pendant 1 mois.

**Tableau 4 détaillant les pourcentages d'aire des impuretés dans les solutions comparatives 1, 2 et invention stockées à 50°C sous 75% d'humidité relative**

| | Comparative 1 | Comparative 2 | Invention |
|---|---|---|---|
| somme des impuretés | 1,35% (7 impuretés) | 1,18% (5 impuretés dont une supérieure à 0,2%) | 1,12% (6 impuretés) |
| principale impureté inconnue | 0,23% | 0,35% | 0,24% |

Au vu des résultats détaillés dans le tableau 4, on relève que la solution selon l'invention présente le moins d'impuretés : 1,12% contre 1,18% et 1,35% (1,35 étant le pourcentage d'impuretés du produit de référence).

Ainsi, la solution selon l'invention présente une meilleure stabilité que la solution comparative 1 que cela soit à 40°C ou à 50°C.

De plus, en comparaison avec la solution comparative 2, la solution selon l'invention présente une nettement meilleure stabilité dans les conditions plus drastiques à savoir à 50°C ; ce qui est très avantageux.

Ainsi, ces résultats témoignent que l'association des deux antioxydants que sont l'EDTA disodique avec la rongalite dans une solution de phosphate de sodium d'hydrocortisone permet d'obtenir des solutions pharmaceutiques selon l'invention parfaitement stables au cours du temps. En effet, les solutions pharmaceutiques selon l'invention présentent un niveau d'impuretés inférieur au produit de référence qu'est l'Efcortesol ®.

De plus, au bout de 6 mois de stockage à 40°C, le pourcentage d'aire de la somme des impuretés de la solution selon l'invention était de 1,65%. Ce résultat est tout à fait satisfaisant, étant donné que la spécification maximale est de 2% et que le phosphate de sodium d'hydrocortisone est également très sensible à la température.

Le tableau 5 ci-dessous détaille les pourcentages d'aire de la somme des impuretés en fonction du temps de la solution selon l'invention qui a été stockée à 25°C sous une humidité relative de 60%.

**Tableau 5 détaillant les pourcentages d'aire des impuretés dans la solution selon l'invention stockées à 25°C sous 60% d'humidité relative en fonction du temps**

| Temps (mois) | 0 | 1 | 2 | 3 | 6 |
|---|---|---|---|---|---|
| somme des impuretés | 0,04% | 0,04% | 0,05 | 0,11 | 0,16 |

Au vu des résultats détaillés dans le tableau 5, on relève que le profil d'impuretés de la solution selon l'invention est parfait, comparativement à une spécification maximale de 2%.

Ces résultats expérimentaux témoignent que les solutions pharmaceutiques d'hydrocortisone selon l'invention sont stables au cours du temps, et ce même dans des conditions forcées (c'est-à-dire avec une hygrométrie élevée, de 60% ou 75% d'humidité relative, et à des températures élevées de 40°C ou 50°C).

## Revendications

1. Solution pharmaceutique d'hydrocortisone qui comprend au moins :
- de l'hydrocortisone ou un sel pharmaceutiquement acceptable de celle-ci ;
- de la rongalite ;
- de l'éthylène-diamine-tétra-acétate disodique (EDTA disodique) ;
- un solvant.

2. Solution pharmaceutique selon la revendication 1, **caractérisée en ce que** le sel pharmaceutiquement acceptable de l'hydrocortisone est choisi parmi le phosphate de sodium d'hydrocortisone, le sodium succinate d'hydrocortisone, l'hydrogéno-succinate d'hydrocortisone, le butyrate d'hydrocortisone et l'acétate d'hydrocortisone.

3. Solution pharmaceutique selon la revendication 2, **caractérisée en ce que** le sel pharmaceutiquement acceptable de l'hydrocortisone est le phosphate de sodium d'hydrocortisone.

4. Solution pharmaceutique selon l'une quelconque des revendications 1 à 3, **caractérisée en ce qu'**elle comprend en outre au moins un tampon.

5. Solution pharmaceutique selon la revendication 4, **caractérisée en ce que** le tampon est choisi parmi l'acétate de sodium, le citrate de sodium, le dihydrogénophosphate de sodium et l'hydrogénophosphate de sodium.

6. Solution pharmaceutique selon l'une quelconque des revendications 1 à 5, **caractérisée en ce que** le pH de la solution pharmaceutique est compris entre 7 et 9, de préférence entre 7,5 et 8,5.

7. Solution pharmaceutique selon l'une quelconque des revendications 1 à 6, **caractérisée en ce que** la concentration en hydrocortisone dans ladite solution pharmaceutique est comprise entre 150 mg/mL et 170 mg/mL.

8. Solution pharmaceutique selon l'une quelconque des revendications 1 à 7, **caractérisée en ce qu'**elle comprend en g pour 200 mL de solution :
- entre 20 g et 60 g, de préférence entre 40 g et 45 g, d'hydrocortisone ou d'un sel pharmaceutiquement acceptable de celle-ci ;
- entre 0,2 g et 1,2 g, de préférence entre 0,3 g et 0,5 g, de rongalite ;
- entre 0,1 g et 0,6 g, de préférence entre 0,15 g et 0,25 g, d'EDTA disodique ;
- entre 160 g et 200 g, de solvant, de préférence d'eau pour préparation injectable.

9. Procédé de préparation de la solution pharmaceutique selon l'une quelconque des revendications 1 à 3, **caractérisé en ce qu'**il comprend au moins les étapes suivantes :
a) on prépare sous agitation un mélange comprenant au moins le solvant, la rongalite et l'EDTA disodique ;
b) on y ajoute sous agitation de l'hydrocortisone ou un sel pharmaceutiquement acceptable de celle-ci de manière à obtenir ladite solution pharmaceutique ;
c) optionnellement on effectue au moins une étape de filtration sur la solution pharmaceutique obtenue à l'issue de l'étape b).

10. Solution pharmaceutique selon l'une quelconque des revendications 1 à 8 pour son utilisation dans le traitement de l'insuffisance surrénalienne aigüe et de l'asthme.

11. Solution pharmaceutique selon la revendication 10, **caractérisée en ce que** ladite solution est administrée par voie parentérale, de préférence par voie intramusculaire.

12. Kit d'injection, de préférence kit d'injection par voie intramusculaire, comportant :
- un dispositif d'injection ;
- la solution pharmaceutique selon l'une quelconque des revendications 1 à 8.

13. Kit d'injection selon la revendication 12, caractérisé en le dispositif d'injection est un dispositif d'injection sans aiguille à cartouche pyrotechnique.

## Patentansprüche

1. Pharmazeutische Hydrocortison-Lösung, die mindestens Folgendes umfasst:
- Hydrocortison oder ein pharmazeutisch verträgliches Salz desselben;
- Rongalit;
- Dinatrium-Ethylendiamintetraacetat (Dinatrium-EDTA);
- ein Lösungsmittel.

2. Pharmazeutische Lösung nach Anspruch 1, **dadurch gekennzeichnet, dass** das pharmazeutisch verträgliche Salz von Hydrocortison aus Hydrocortison-Natriumphosphat, Hydrocortisonsuccinat-Natrium, Hydrocortison-Hydrogensuccinat, Hydrocortisonbutyrat und Hydrocortisonacetat ausgewählt wird.

3. Pharmazeutische Lösung nach Anspruch 2, **dadurch gekennzeichnet, dass** das pharmazeutisch verträgliche Salz von Hydrocortison Hydrocortison-Natriumphosphat ist.

4. Pharmazeutische Lösung nach einem der Ansprüche 1 bis 3, **dadurch gekennzeichnet, dass** sie weiter mindestens einen Puffer umfasst.

5. Pharmazeutische Lösung nach Anspruch 4, **dadurch gekennzeichnet, dass** der Puffer aus Natriumacetat, Natriumcitrat, Natriumdihydrogenphosphat und Natriumhydrogenphosphat ausgewählt wird.

6. Pharmazeutische Lösung nach einem der Ansprüche 1 bis 5, **dadurch gekennzeichnet, dass** der pH-Wert der pharmazeutischen Lösung zwischen 7 und 9 und vorzugsweise zwischen 7,5 und 8,5 enthalten ist.

7. Pharmazeutische Lösung nach einem der Ansprüche 1 bis 6, **dadurch gekennzeichnet, dass** die Hydrocortison-Konzentration in der pharmazeutischen Lösung zwischen 150 mg/ml und 170 mg/ ml enthalten ist.

8. Pharmazeutische Lösung nach einem der Ansprüche 1 bis 7, **dadurch gekennzeichnet, dass** sie, in g, für 200 ml Lösung Folgendes umfasst:
- zwischen 20 g und 60 g, vorzugsweise zwischen 40 g und 45 g an Hydrocortison oder einem pharmazeutisch verträglichen Salz desselben;
- zwischen 0,2 g und 1,2 g, vorzugsweise zwischen 0,3 g und 0,5 g an Rongalit;
- zwischen 0,1 g und 0,6 g, vorzugsweise zwischen 0,15 g und 0,25 g an Dinatrium-EDTA;
- zwischen 160 g und 200 g an Lösungsmittel, vorzugsweise Wasser für eine injizierbares Präparat.

9. Verfahren zur Herstellung der pharmazeutischen Lösung nach einem der Ansprüche 1 bis 3, **dadurch gekennzeichnet, dass** es mindestens die folgenden Schritte umfasst:
a) Herstellen, unter Schütteln, eines Gemisches, das mindestens das Lösungsmittel, Rongalit und Dinatrium-EDTA umfasst;
b) Beigeben, unter Schütteln, von Hydrocortison oder von einem pharmazeutisch verträglichen Salz desselben, um die pharmazeutische Lösung zu erhalten;
c) optional Durchführen mindestens eines Filterungsschrittes an der am Ende des Schrittes b) erhaltenen pharmazeutischen Lösung.

10. Pharmazeutische Lösung nach einem der Ansprüche 1 bis 8, für deren Verwendung bei der Behandlung einer akuten Nebenniereninsuffizienz und von Asthma.

11. Pharmazeutische Lösung nach Anspruch 10, **dadurch gekennzeichnet, dass** die Lösung parenteral, vorzugsweise intramuskulär verabreicht wird.

12. Injektionsset, vorzugsweise intramuskuläres Injektionsset, Folgendes beinhaltend:
- eine Injektionsvorrichtung;
- die pharmazeutische Lösung nach einem der Ansprüche 1 bis 8.

13. Injektionsset nach Anspruch 12, **dadurch gekennzeichnet, dass** die Injektionsvorrichtung eine nadellose Injektionsvorrichtung mit pyrotechnischer Kartusche ist.

## Claims

1. An hydrocortisone pharmaceutical solution which comprises at least:
- hydrocortisone or a pharmaceutically acceptable salt thereof;
- rongalite;
- disodium ethylenediaminetetraacetic (disodium EDTA);
- a solvent.

2. The pharmaceutical solution according to claim 1, **characterized in that** the pharmaceutically acceptable salt of hydrocortisone is selected from hydrocortisone sodium phosphate, hydrocortisone sodium succinate, hydrocortisone hydrogen succinate, hydrocortisone butyrate and hydrocortisone acetate.

3. The pharmaceutical solution according to claim 2, **characterized in that** the pharmaceutically acceptable salt of hydrocortisone is the hydrocortisone sodium phosphate.

4. The pharmaceutical solution according to any one of claims 1 to 3, **characterized in that** it further comprises at least one buffer.

5. The pharmaceutical solution according to claim 4, **characterized in that** the buffer is selected from sodium acetate, sodium citrate, sodium dihydrogen phosphate and sodium hydrogen phosphate.

6. The pharmaceutical solution according to any one of claims 1 to 5, **characterized in that** the pH of the pharmaceutical solution is comprised between 7 and 9, preferably between 7.5 and 8.5.

7. The pharmaceutical solution according to any one of claims 1 to 6, **characterized in that** the concentration of hydrocortisone in said pharmaceutical solution is comprised between 150 mg/mL and 170 mg/mL.

8. The pharmaceutical solution according to any one of claims 1 to 7, **characterized in that** it comprises in g per 200 mL of solution:
- between 20 g and 60 g, preferably between 40 g and 45 g, of hydrocortisone or a pharmaceutically acceptable salt thereof;
- between 0.2 g and 1.2 g, preferably between 0.3 g and 0.5 g, of rongalite;
- between 0.1 g and 0.6 g, preferably between 0.15 g and 0.25 g, of disodium EDTA;
- between 160 g and 200 g of solvent, preferably of water for injectable preparation.

9. A method for preparing the pharmaceutical solution according to any one of claims 1 to 3, **characterized in that** it comprises at least the following steps:
a) preparing under stirring a mixture comprising at least the solvent, the rongalite and the disodium EDTA;
b) adding under stirring the hydrocortisone or a pharmaceutically acceptable salt thereof so as to obtain said pharmaceutical solution;
c) optionally performing at least one filtration step on the pharmaceutical solution obtained at the end of step b).

10. The pharmaceutical solution according to any one of claims 1 to 8 for its use in the treatment of acute adrenal insufficiency and asthma.

11. The pharmaceutical solution according to claim 10, **characterized in that** said solution is parenterally, preferably intramuscularly administered.

12. An injection kit, preferably an intramuscular injection kit, including:
- an injection device;
- the pharmaceutical solution according to any one of claims 1 to 8.

13. The injection kit according to claim 12, **characterized in that** the injection device is a needleless injection device with pyrotechnic cartridge.
